# EUROPEAN PATENT APPLICATION

(11) **EP 4 239 054 A1**
(43) Date of publication of application: **06.09.2023**
(21) Application number: 21886339.7
(22) Date of filing: 28.10.2021
(51) Int. Cl.: C12M 3/00, C12N 5/071, C12N 5/079, A61L 27/38

(54) **ARTIFICIAL AXON BUNDLE**

(30) Priority: 29.10.2020 JP 2020181354
(71) Applicant: Keio University, Tokyo, 108-8345 (JP); Jiksak Bioengineering, Inc., Kawasaki-shi, Kanagawa, 212-0032 (JP)
(72) Inventor: SHIBATA, Shinsuke, Tokyo 160-8582 (JP); KIMURA, Hiroo, Tokyo 160-8582 (JP); NISHIJIMA, Takayuki, Tokyo 160-8582 (JP); SHINDO, Tomoko, Tokyo 160-8582 (JP); OKANO, Hideyuki, Tokyo 160-8582 (JP); KAWADA, Jiro, Kawasaki-shi, Kanagawa 212-0032 (JP); TOITA, Sayaka, Kawasaki-shi, Kanagawa 212-0032 (JP)
(74) Representative: J A Kemp LLP
(86) International application number: PCT/JP2021/039869
(87) International publication number: WO 2022/092222

(57) **Abstract**

The present invention relates to an axon bundle obtained by a method of culturing a nerve cell aggregation having one spheroid and an axon bundle extending from the spheroid
wherein the nerve cell aggregation consisting of a plurality of neurons having a cell body and an axon obtained by a method comprising the following step:
supplying a culture medium to one first chamber, one second chamber and one channel having the length of at least 1 mm, the width of 100 to 150 µm and the hight of 100 to 200 µm, which connecting said first chamber and second chamber, wherein said first chamber, second chamber, and channel are contained in one of the modules disposed in a culture plate,
seeding the first chamber with a nerve cell derived from a stem cell or a spheroid of primary cultured nerve cells; and
culturing said nerve cell, thereby growing an axon bundle from said spheroid and
extending them into said channel,
wherein the length of the axon bundle obtained is 1 mm or more, and the diameter of the axon bundles is 50 µm or more.

## Description

### TECHNICAL FIELD

This invention relates to artificial axon bundles, bundles of artificial axon bundles, artificial nerves, and nerve grafts.

### BACKGROUND ART

After trauma with extensive contusion or extensive resection for tumors, functional reconstruction of the defective nerve may be necessary. When the defect is small, end-to-end suture has been used, and when the defect is large, autologous nerve grafting has been used. However, there are problems such as the difficulty of application for defects larger than 40 mm, the limited length and amount of nerve that can be harvested, and the long time required for the surgery.

Another known method is to use nerve regeneration induction tubes, so-called artificial nerves. However, clinical outcomes are said to deteriorate when the length of the tube exceeds 20 mm.

Furthermore, decellularized allogeneic nerve grafts have already been approved by the FDA (U.S. Food and Drug Administration) and have been used in the United States. However, even with allogeneic nerve grafts, it is believed that autologous nerve grafts should be chosen when the allogeneic nerve graft exceeds 30 mm in length. In addition, there are concerns about unknown infectious diseases.

On the other hand, a culture device capable of rapidly growing axon bundles extending from neurons in vitro and a method of culturing axon bundles using this device have been developed (Patent Literature 1).

### CITATION LIST

### Patent Literature

Patent Literature 1: WO2017/187696

### SUMMARY OF INVENTION

### Technical Problem

After diligent research, the inventor has surprisingly developed an artificial axon bundle that can be applied to a wide range of nerve defects by using an axon bundle culture method, thus completing the invention.

### Solution to Problem

A summary of the present invention is as described below.
[1] An axon bundle obtained by a method of culturing a nerve cell aggregation having one spheroid and an axon bundle extending from the spheroid wherein the nerve cell aggregation consisting of a plurality of neurons having a cell body and an axon obtained by a method comprising the following step:
   supplying a culture medium to one first chamber, one second chamber and one channel having length of at least 1 mm, width of 100 to 150 µm and hight of 100 to 200 µm, which connecting said first chamber and second chamber, wherein said first chamber, second chamber, and channel are contained in one of the modules disposed in a culture plate,
   seeding the first chamber with a nerve cell derived from a stem cell or a spheroid of primary cultured nerve cells; and
   culturing said nerve cell, thereby growing an axon bundle from said spheroid and extending them into said channel,
   wherein the length of the axon bundle obtained is 1 mm or more, and the diameter of the axon bundles is 50 µm or more.
[2] The axon bundle according to [1], wherein the stem cells are pluripotent stem cells or iPS derived from neural stem cell.
[3] A bundle of axon bundles bundling of two or more axon bundles according to [1] or [2] by using tissue adhesive.
[4] An artificial nerve comprising a bundle of axon bundles according to [3].
[5] The bundle of axon bundles according to [3] or the artificial nerve according to [4], wherein the diameter is 1 mm or more.
[6] The artificial nerve according to [4] or [5], for transplantation into a living body.
[7] The artificial nerve according to [4] or [5], for promoting regeneration of a cut nerve.
[8] A nerve graft, wherein the artificial nerve according to any one of [4] to [7] is introduced into a medically acceptable hollow tubular structure.

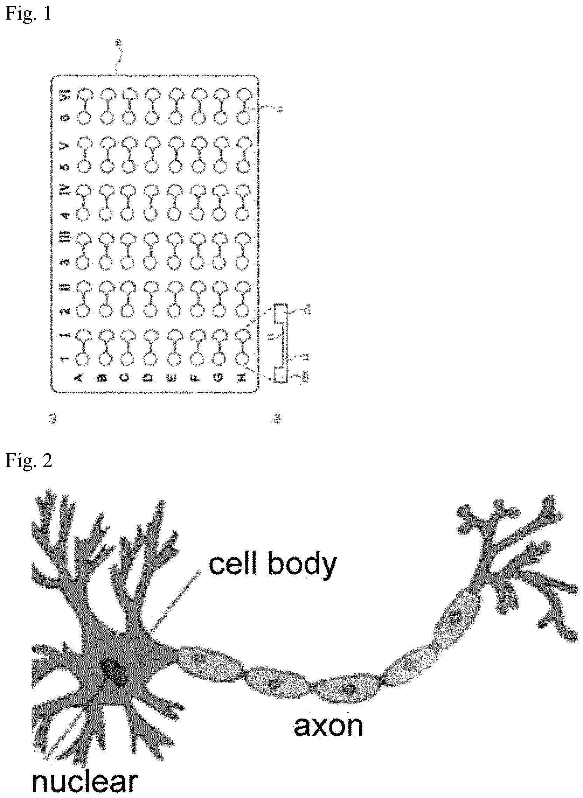

Each of the first and second chambers 12a and 12b is a cylindrical recess and is formed as a well-shaped depression whose bottom is closed by the substrate 15 and whose top is open (Fig. 1). One end of the channel 13 opens to the lower end of the side wall of the first chamber 12a, and the other end opens to the lower end of the side wall of the second chamber 12b. The width of the channel 13 is preferably 100 to 150 [µm] and the height is preferably 100 to 200 [µm], but these dimensions are not limited to such numerical values. It can be adjusted as needed. The insides of the first and second chambers 12a and 12b and the channel 13 are filled with the culture solution. Since the structure of the module 11 is simple, the culture solution can smoothly flow into the module 11. Also, since the size of the channel 13 is larger than in conventional devices, the culture fluids in the first and second chambers 12a, 12b can naturally mix. The planar shape of each of the first and second chambers 12a and 12b is preferably circular but is not limited to circular and it can be changed as necessary. Any conventionally known culture medium suitable for culturing nerve cells can be used as the culture medium. For example, DMEM (Dulbecco's Modified Eagle's Medium) or Neurobasal medium is used as a basal medium, supplemented with a supplement such as N2 or B27, and neurotrophic factors such as BDNF (Brain-derived neurotrophic factor).

Nerve cells are seeded inside one of the first and second chambers 12a, 12b. Either of the first and second chambers 12a and 12b can be selected as the chamber for seeding the cells. The seeded cell number and cell density can be set as appropriate. Neuronal spheroids can also be seeded. After culturing, the axons of the nerve cells extend through the channel 13 to the second chamber 12b and form bundles within the channel 13 . The inner diameter of channel 13 is desirably large enough to accommodate an axonal bundle of nerve cells.

Stem cells are cells that have the ability (self-renewal ability) to divide and create the same cells as themselves and the ability to differentiate into different types of cells and can proliferate endlessly. Pluripotent stem cells include embryonic stem cells (ES cells) and induced pluripotent stem cells (iPS cells). Neural stem cells are oligopotent stem cells. These can be induced to differentiate into nerve cells. A nerve cell derived from a stem cell is a nerve cell that is induced to differentiate from a stem cell into a nerve cell. Contamination with pathogens can be avoided by using nerve cells derived from stem cells.

Primary cultured neurons are cell cultures directly using animal tissues. Primary cultured cells are considered to maintain a state like that in vivo and are therefore used in various studies. As for cells constituting nerve tissue, most nervous system cells such as neurons (nerve cells) and glial cells (glial cells) such as astrocytes, oligodendrocytes, Schwann cells, and microglia can be primary cultured.

A spheroid is a spherical cell aggregate in which cells aggregate. For example, it can be produced by three-dimensional culture using hydrogel.

A nerve cell is a cell that constitutes each nervous system of the central nervous system or the peripheral nervous system, and in the present invention, a whole cell is used synonymously with a neuron. Neurons are divided into three main parts: the cell body with the nucleus, the dendrites that receive input from other cells, and the axons that output to other cells (Fig. 2). A cell body (soma) is a site where cell organelles such as cell nuclei are concentrated in nerve cells and dendrites and axons are associated. Axons are projection-like structures that extend from the cell body and are responsible for the output of signals in nerve cells. It is the part of a nerve cell that varies greatly in length, ranging from a few millimeters in humans for connecting adjacent cells to tens of centimeters extending into the spinal cord. Basically, only one axon extends from one cell body. Nerve cells include motor neurons, sensory neurons, and autonomic neurons.

In vivo, fasciculate structures of numerous axons connecting distant cell populations are often observed). For example, motor neurons, which transmit commands from the cerebrum to muscles, extend fasciculated axons (axonal bundles) from the spinal cord toward the muscles. In addition, the cerebral cortex is divided into regions consisting of different neuronal groups for each function such as motor control, language processing, and visual information processing, and these regions are connected by axonal fascicles.. Artificial axonal bundles are useful for functional reconstruction of such nerve damage.

The length of the axon bundle is 1 mm or longer, 5 mm or longer, 10 mm or longer, 20 mm or longer, 30 mm or longer, 40 mm or longer, 50 mm or longer, or 100 mm or longer. The diameter of the axonal bundle is 10 µm or greater, 25 µm or greater, 50 µm or greater, or 100 µm or greater. The length of the axonal bundle can be measured as the length of the axonal bundle on the image, for example, by image analysis of the axonal bundle photographed using an optical microscope or a digital camera. The diameter of the axonal bundle can also be measured as the minimum thickness of the axonal bundle on the image, by image analysis of the axonal bundle photographed using an optical microscope or an electron microscope. In addition, the diameter of the axon bundle is 80 µm on average, and the maximum width (150 µm) is equivalent to that of the channel 13 .

The axonal bundles of the present invention can be bundled using tissue adhesives. Tissue adhesives are composed of proteins and bioabsorbable materials, and examples thereof include collagen and plasma derivatives. In the present invention, bundled axonal bundles are referred to as "bundle of axonal bundles".

A bundle of axonal bundles can be used as an artificial nerve. Artificial nerves can be implanted in living organisms and used to promote regeneration of damaged nerves. Nerve prostheses can include tissue adhesives and the like. The diameter of bundle of the axonal bundles is 0.5 mm or greater, 1 mm or greater, 2 mm or greater, 5 mm or greater, 10 mm or greater, or 20 mm or greater. Preferably, the diameter of bundle of the axonal bundles is 1 mm or more. The diameter of the artificial nerve is 1 mm or more, 2 mm or more, 4 mm or more, 10 mm or more, 20 mm or more, or 40 mm or more. Preferably, the artificial nerve has a diameter of 1 mm or more.

The artificial nerve of the present invention can also be introduced into medically acceptable hollow tubular structures and used as nerve grafts. Examples of medically acceptable materials for hollow tubular structures include silicone tubes and collagen tubes. The inner diameter of the hollow tubular structure can be 1 mm. The outer diameter of the hollow tubular structure can be 2 mm. The thickness of the hollow tubular structure can be 1 mm.

### Effects of Invention

The artificial axon bundle, bundle of artificial axon bundles, artificial nerves, and nerve grafts of the present invention can be applied to a wide range of nerve defects, including motor and sensory nerves, to regenerate the function of the defective nerves.

### BRIEF DESCRIPTION OF DRAWINGS

[Fig. 1] Fig. 1 represents a culture plate.
[Fig. 2] Fig. 2 is an illustration of a nerve cell.

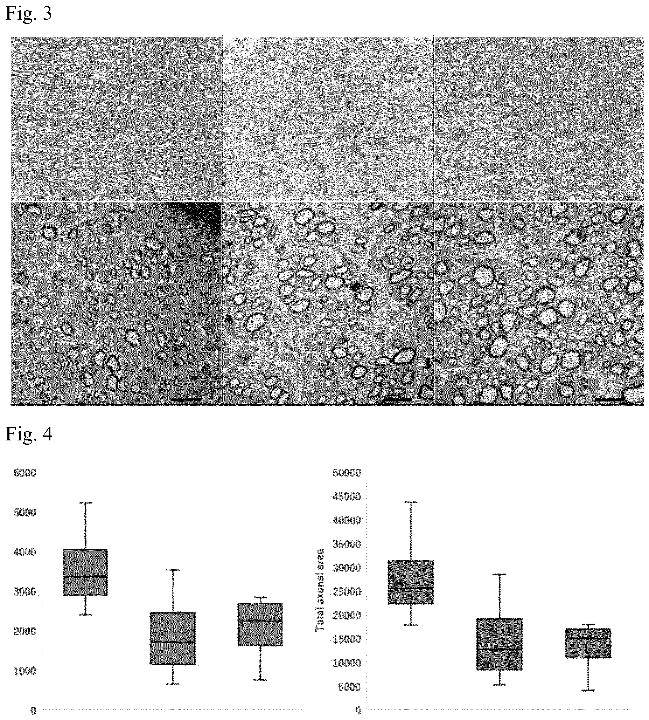

[Fig. 3] FIG. 3 shows histological evaluation by observation of regenerated nerve tissue. The horizontal axis represents, from the left, the collagen gel-implanted group (n=7), the group of 6 axonal bundles (n=6), and the group of 12 axonal bundles (n=6). The lower part is an enlarged view of the upper part. Scale bars are 10 µm each. In all samples of the axon graft transplantation group, a larger number of thicker axons were regenerated than in the control group.
[Fig. 4] Fig. 4 represents histological evaluation by quantitative analysis of regenerating axonal area. The figure on the left shows the number of regenerating axons. The vertical axis is the number of axons, and the horizontal axis is 12 bundles of artificial axons, 6 bundles of artificial axons, and NC from the left. The figure on the right is the total area of the regenerating axonal area. The vertical axis is the total axonal area (µm² ), and the horizontal axis is 12 bundles of artificial axons, 6 bundles of artificial axons, and NC from the left.

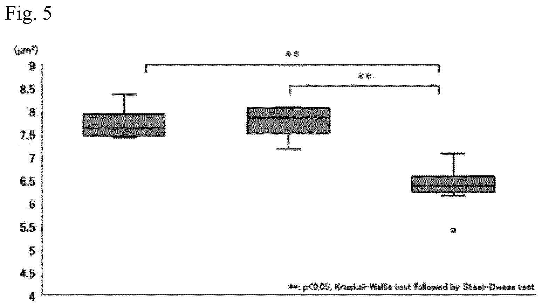

[Fig. 5] Fig. 5 represents histological evaluation by quantitative analysis of regenerating axonal area. The vertical axis is the total axon area (µm² ) per number of axons, and the horizontal axis is 12 bundles of artificial axons, 6 bundles of artificial axons, and NC from the left. The mean axonal area of the axonal graft army was significantly larger than that of the control group, and thick nerves were regenerated (p<0.05, Kruskal-Wallis test, followed by Steeldwas test).

### Description of Embodiments

The invention is illustrated in detail by the following examples.

### EXAMPLES

Motor neurons differentiated from human iPS cells are seeded in one or two cylindrical recesses in the culture chamber shown in FIG. It was cultured in an incubator at 37° C. and 5 vol % carbon dioxide for months. The composition of the culture solution was prepared by adding GDNF 20 ng/ml, BDNF 50 ng/ml, B27 Plus supplement 1:50, and Penicillin-Streptomycin (Thermofisher Scientific) to the basal medium, Neurobasal plus medium (Thermofisher Scientific).

Neurites extended from the structure of the culture chamber into channels extending from within the culture chamber. The cell bodies remained in the culture chamber, forming neuroaxon bundles with neurites longer than 2 cm but without cell bodies in the channels. The single axon bundle contained at least several hundred axons, depending greatly on the distance from the cell body and the number of culture days.

Nerve axon bundles were cultured by changing the composition of the culture medium in the range of 1 to 200 ng/ml for GDNF and 1 to 200 ng/ml for BDNF. The length and diameter of the obtained nerve axon bundle were measured as follows.

The axon bundle formed in the channel 13 was imaged at 5 times magnification with an optical microscope (Leica microsystems) while being cultured in the channel 13. The diameter of the nerve axon bundle in the captured image was measured as the thickness of the thinnest part of the nerve axon bundle. Also, the length of the nerve axon bundle on the captured image was measured. 10 nerve axon bundles were randomly selected from the nerve axon bundles cultured above, imaged, the diameter and length were calculated, and the average was calculated. As a result, the average diameter of the nerve axon bundle was 80 µm, and the average length was 5 mm. The minimum length of the axonal nerve bundle cultured above was 0.5 mm, the maximum length was 6 mm, the minimum diameter was 10 µm, and the maximum diameter was 150 µm.

Multiple (2-6) axonal bundles were enclosed in a silicone tube (1.5 mm inner diameter, 2.5 mm outer diameter, manufactured by Taiyo Kogyo Co., Ltd.) using collagen gel (Nitta Gelatin), and the ischium of mice and rats. It was implanted into the nerve and evaluated for its functional and histological recovery.

The sciatic nerve injury model was prepared by immunodeficient NOD-SCID mice, wild-type c57bl6j mice (6-week-old, male, Sankyo Lab) and immunodeficient Nude rats and wild-type SD rats (7-week-old, male, Sankyo Lab). used. All mice were deeply anesthetized with a mixed solution of ketamine (100 mg/kg; Sankyo) and xylazine (10 mg/kg; Bayer) by intraperitoneal injection.

Eight weeks after implantation, a longitudinal skin incision was made on the dorsum of the thigh and the sciatic nerve was exposed by incising the thigh and dividing the thigh muscle with a scalpel. Experimental animals were divided into three groups each time: (1) axonal bundle artificial nerve transplantation group; ) A comparison was made between a control negative control group consisting of a collagen gel without axonal bundles and a silicone tube.

In (1) axonal bundle artificial nerve transplantation group and (2) target negative control group, a sciatic nerve defect of about 6 mm was created in mice, and multiple axonal bundles were transplanted with a silicone tube length of 8 mm. The nerve stump was secured 1 mm inside the tube stump using a 9-0 monofilament nylon suture. In rats, a sciatic nerve defect of about 12 mm was created, the length of the silicone tube was 14 mm, and 9-0 monofilament nylon suture was used at both ends to fix the nerve stump 2 mm inside the tube stump.

As a result, (1) the mice and rats in the axonal bundle artificial nerve transplantation group showed functionally excellent motor function recovery compared to the mice and rats in the (3) negative control group. Histologically, a wider area of new nerve tissue regeneration was observed. Since this experiment was evaluated 8 weeks after the transplantation, further effects are expected if more time passes after the transplantation.

### INDUSTRIAL APPLICABILITY

The artificial axon bundle, the bundle of artificial axon bundles, the artificial nerve, and the nerve graft of the present invention can be used for functional reconstruction of damaged nerves.

## Claims

1. An axon bundle obtained by a method of culturing a nerve cell aggregation having one spheroid and an axon bundle extending from the spheroid wherein the nerve cell aggregation consisting of a plurality of neurons having a cell body and an axon obtained by a method comprising the following step:
supplying a culture medium to one first chamber, one second chamber and one channel having the length of at least 1 mm, the width of 100 to 150 µm and the hight of 100 to 200 µm, which connecting said first chamber and second chamber, wherein said first chamber, second chamber, and channel are contained in one of the modules disposed in a culture plate,
seeding the first chamber with a nerve cell derived from a stem cell or a spheroid of primary cultured nerve cells; and
culturing said nerve cell, thereby growing an axon bundle from said spheroid and extending them into said channel,
wherein the length of the axon bundle obtained is 1 mm or more, and the diameter of the axon bundles is 50 µm or more.

2. The axon bundle according to claim 1, wherein the stem cells are pluripotent stem cells or iPS derived from neural stem cell.

3. A bundle of axon bundles bundling of two or more axon bundles according to claim 1 or 2]by using tissue adhesive.

4. An artificial nerve comprising a bundle of axon bundles according to claim 3.

5. The bundle of axon bundles according to claim 3 or the artificial nerve according to claim 4, wherein the diameter is 1 mm or more.

6. The artificial nerve according to claim 4 or 5, for transplantation into a living body.

7. The artificial nerve according to claim 4 or 5, for promoting regeneration of a cut nerve.

8. A nerve graft, wherein the artificial nerve according to any one of claims 4 to 7 is introduced into a medically acceptable hollow tubular structure.
